# EUROPÄISCHE PATENTSCHRIFT

(11) **EP 2 012 688 B1**
(45) Veröffentlichungstag und Bekanntmachung des Hinweises auf die Patenterteilung: **26.08.2015**
(21) Anmeldenummer: 07703578.0
(22) Anmeldetag: 02.03.2007
(51) Int. Cl.: A61B 17/68, A61B 17/82

(54) **Chirurgische Fixiereinrichtung für zwei Knochenteile**
Surgical fixing device for two bone parts
Dispositif chirurgical de fixation de deux parties d'os

(30) Priorität: 28.04.2006 DE 102006021025
(43) Veröffentlichungstag der Anmeldung: 14.01.2009
(73) Patentinhaber: Aesculap AG, 78532 Tuttlingen (DE)
(72) Erfinder: MORALES, Pedro, 78532 Tuttlingen (DE); WEISSHAUPT, Dieter, 78194 Immendingen (DE); DWORSCHAK, Manfred, 78589 Dürbheim (DE); LUTZE, Theodor, 78582 Balgheim (DE)
(74) Vertreter: Hoeger, Stellrecht & Partner Patentanwälte mbB
(86) Internationale Anmeldenummer: PCT/EP2007/001791
(87) Internationale Veröffentlichungsnummer: WO 2007/124807

(56) Entgegenhaltungen:
- WO-A-02/09602
- WO-A-2006/002744
- DE-A1- 10 326 690
- DE-B3-102004 038 823
- JP-A- 2003 220 070

## Beschreibung

Die Erfindung betrifft eine chirurgische Fixiereinrichtung zur gegenseitigen Fixierung von zwei nebeneinanderliegenden Knochenteilen mit einem ersten Anlageelement zur Anlage an der Unterseite der beiden Knochenteile und mit einem zweiten Anlageelement zur Anlage an der Oberseite der Knochenteile und mit einer Spanneinrichtung, die die beiden Anlageelemente durch einen Zwischenraum zwischen den Knochenteilen hindurch miteinander verbindet und die die beiden Anlageelemente dauerhaft gegeneinander gespannt fixiert, wenn sie an der Unterseite bzw. der Oberseite der Knochenteile anliegen, wobei die Fixiereinrichtung mindestens ein elastisches Spannelement umfaßt, welches eine an die Oberseite der Knochenteile anlegbare Stützfläche gegen das erste Anlageelement verschiebt, so daß die Stützfläche und das erste Anlageelement gegen die Kraft des elastischen Spannelementes voneinander entfernbar sind und wobei die Stützfläche Teil des elastischen Spannelementes ist.

Eine solche Fixiereinrichtung ist beispielsweise aus der WO 02/09602 A1 bekannt. Mit derartigen Fixiereinrichtungen können zwei nebeneinander liegende Knochenteile, beispielsweise zwei Knochenteile des Schädelknochens oder des Brustbeines, seitlich nebeneinander liegend fixiert werden, wobei zwischen den beiden Knochenteilen ein sehr schmaler Zwischenraum verbleibt, durch den hindurch die beiden Anlageelemente gegeneinander gespannt werden. Weitere Fixiervorrichtungen zur gegenseitigen Fixierung von zwei nebeneinanderliegenden Knochenteilen sind insbesondere in der DE 103 26 690 B4, DE 10 2004 038 823 B3, der WO 2006/002744 A1 und der JP 2003 220070 A offenbart.

Schwierig ist das Anlegen einer solchen Vorrichtung, da einerseits die beiden Anlageelemente an der Unterseite und an der Oberseite der Knochenteile positioniert werden müssen und da auch andererseits die beiden Knochenteile entsprechend zusammengeführt werden müssen. Insbesondere bei Verwendung von Anlageelementen, die in einer bestimmten Richtung implantiert werden müssen, beispielsweise bei länglichen, rechteckigen Anlageelementen, kann dies zu Problemen führen.

Es ist Aufgabe der Erfindung, eine chirurgische Fixiereinrichtung der gattungsgemäßen Art so auszubilden, daß das Anlegen der Fixiereinrichtung erleichtert wird.

Diese Aufgabe wird bei einer chirurgischen Fixiereinrichtung der eingangs beschriebenen Art erfindungsgemäß dadurch gelöst, daß das elastische Spannelement mit der Fixiereinrichtung lösbar verbunden ist.

Eine solche Ausgestaltung macht es möglich, die chirurgische Fixiereinrichtung vor dem Zusammenführen der Knochenteile an einem der beiden Knochenteile so festzulegen, daß das Anlageelement die richtige Position einnimmt, die Knochenteile aber noch nicht mit der endgültigen Kraft gegeneinander spannt. Durch das elastische Spannelement wird bei diesem ersten Anlegen der Fixiereinrichtung an einem der beiden Knochenteile das erste Anlageelement an die Unterseite des Knochenteiles gezogen, während sich an die Oberseite dieses Knochenteiles die Stützfläche elastisch anlegt. Die Stützfläche und das erste Anlageelement nehmen damit das erste Knochenteil elastisch zwischen sich auf und halten die Fixiereinrichtung in dieser Position am ersten Knochenteil. Es ist dann möglich, das zweite Knochenteil seitlich zwischen das erste Anlageelement und die Stützfläche einzuschieben, gegebenenfalls unter elastischer Aufweitung des Zwischenraums zwischen dem ersten Anlageelement und der Stützfläche, und erst dann werden das erste Anlageelement und das zweite Anlageelement endgültig gegeneinander gespannt. Es ist vorteilhaft, daß die Stützfläche Teil des elastischen Spannelementes und das elastische Spannelement mit der Fixiereinrichtung lösbar verbunden ist. Nach dem ersten Anlegen der Fixiereinrichtung und nach dem Einschieben des zweiten Knochenteils zwischen erstes Anlageelement und Stützfläche ist es daher möglich, das elastische Spannelement zu entfernen und dann die beiden Anlageelemente endgültig gegeneinander zu spannen. Die chirurgische Fixiereinrichtung bleibt auch nach der Entfernung des elastischen Spannelementes in ihrer Position, sei es dadurch, daß sich das erste Anlageelement in die anliegenden Knochenteile eingegraben hat und dadurch fixiert ist, sei es dadurch, daß der Operateur die Fixiereinrichtung durch ein geeignetes Handhabungsinstrument an der Spanneinrichtung festhält.

Dabei kann insbesondere vorgesehen sein, daß die Stützfläche vor dem Anlegen der Fixiereinrichtung an die Knochenteile zwischen den beiden Anlageelementen angeordnet ist.

Das elastische Spannelement kann besonders vorteilhaft an der Spanneinrichtung gehalten sein.

Beispielsweise kann bei einer bevorzugten Ausführungsform vorgesehen werden, daß die Spanneinrichtung mindestens einen am ersten Anlageelement gehaltenen und das zweite Anlageelement durchsetzenden Stab aufweist und daß das elastische Spannelement an diesem Stab gehalten ist.

insbesondere kann es dabei am freien Ende dieses Stabes gehalten sein.

Bei einer abgewandelten Ausführungsform weist die Spanneinrichtung zwei nebeneinander angeordnete Stäbe auf und das elastische Spannelement umfaßt eine die beiden Stäbe verbindende Brücke.

Weiterhin kann das Spannelement mindestens ein Klemmelement tragen, welches das Spannelement an dem Stab oder den Stäben lösbar festlegt. Das Klemmelement hält das elastische Spannelement so an dem Stab oder an den Stäben, daß das elastische Gegeneinanderspannen von Stützfläche einerseits und erstem Anlageelement andererseits gewährleistet ist, trotzdem ist die Klemmkraft jedoch nur so groß, daß das elastische Spannelement von dem Stab oder den Stäben abgezogen werden kann, wenn die Anlageelemente gegeneinander gespannt werden sollen.

Bei einer bevorzugten Ausführungsform weist das Spannelement einen die Stützfläche und die Verbindungsstelle mit der Spanneinrichtung verbindenden, seitlich an dem zweiten Anlageelement vorbei geführten Federarm auf.

Das Spannelement kann ein die Spanneinrichtung überragendes Griffstück tragen, so daß der Operateur das elastische Spannelement an diesem Griffstück ergreifen und gegebenenfalls vor dem Spannen der Anlageelemente das elastische Spannelement entfernen kann.

Bei einer weiteren bevorzugten Ausführungsform ist vorgesehen, daß sich das elastische Spannelement an dem zweiten Anlageelement abstützt.

Insbesondere kann das elastische Spannelement bei an die Knochenteile angelegter Fixiereinrichtung seitlich zwischen die Oberseite der Knochenteile und die Unterseite des zweiten Anlageelementes eingeschoben und seitlich wieder herausschiebbar sein. In diesem Falle ist also das elastische Spannelement nicht dauerhaft mit der Fixiereinrichtung verbunden, sondern als separates Teil ausgebildet, das zwischen das zweite Anlageelement und die Oberseite der Knochenteile eingeschoben wird und dadurch die Fixiereinrichtung federnd in der Anlageposition hält.

Insbesondere kann das elastische Spannelement eine Bügelfeder sein, es wäre aber auch möglich, bei allen Ausführungsformen andere Federtypen zu verwenden, beispielsweise Schraubenfedern, Tellerfedern, Polster aus elastischem Material, etc.

Besonders vorteilhaft ist es, wenn die Bügelfeder U-förmig ausgebildet ist mit einem die Stützfläche bildenden ersten Arm, einem an dem zweiten Anlageelement anlegbaren zweiten Arm und mit einem die beiden Arme verbindenden federnden Steg.

Eine besonders günstige Anordnung ergibt sich, wenn die Spanneinrichtung mindestens einen am ersten Anlageelement gehaltenen und das zweite Anlageelement durchsetzenden Stab aufweist und wenn das Spannelement auf beiden Seiten des Stabes oder der Stäbe angeordnet ist. Auf diese Weise ergibt sich in gleicher Weise eine elastische Vorfixierung der Fixiereinrichtung unabhängig davon, ob auf der linken Seite oder auf der rechten Seite der Fixiereinrichtung das entsprechende Knochenteil eingeschoben wird.

Das elastische Spannelement kann beispielsweise eine schlitzförmige Ausnehmung aufweisen, in die der oder die Stäbe der Spanneinrichtung beim Einschieben des elastischen Spannelements eintreten. Dadurch erhält man gleichzeitig eine Ausrichtung des elastischen Spannelementes, da die schlitzförmige Ausnehmung als Führung für das elastische Spannelement wirkt.

Günstig ist es, wenn mindestens ein Arretierglied vorgesehen ist, welches das elastische Spannelement im eingeschobenen Zustand gegen ein Herausschieben sichert, und wenn das Arretierglied durch einen Grenzwert überschreitende Verschiebekräfte lösbar ist. Damit wird das eingeschobene elastische Spannelement in der eingeschobenen Stellung zwar fixiert, kann aber durch eine relativ große Kraft aus der Anlageposition herausgezogen werden. Beispielsweise kann das Arretierglied durch ineinandergreifende Vor- und Rücksprünge an dem elastischen Spannelement und an dem zweiten Anlageelement gebildet werden.

Es ist weiterhin vorteilhaft, wenn die Stützfläche an ihrer beim Herausziehen vorderen Kante nach oben ansteigend geformt ist. Das erleichtert das Herausziehen des elastischen Spannelementes, da die nach oben ansteigende, beispielsweise nach oben umgebogene Kante als Aufgleitfläche wirkt.

In ähnlicher Weise können an den Seitenkanten der Stützflächen Aufgleitflächen angeordnet sein, die das Einschieben der Knochenteile zwischen Stützfläche und erstes Anlageelement erleichtern.

Auch bei einem in dieser Weise lösbaren elastischen Spannelement ist es günstig, wenn dieses ein im eingeschobenen Zustand des elastischen Spannelementes seitlich über das zweite Anlageelement vorstehendes Griffstück trägt. Dieses dient zum Ergreifen des Spannelementes und an diesem Griffstück kann das Spannelement aus seiner Spannstellung zwischen den Knochenteilen und dem zweiten Anlageelement herausgezogen werden.

Bei einer weiteren bevorzugten, jedoch nicht erfindungsgmäßen Ausführungsform ist vorgesehen, daß die Stützfläche durch das zweite Anlageelement gebildet wird.

In diesem Falle muß das zweite Anlageelement durch das elastische Spannelement gegen das erste Anlageelement gespannt werden, und auch dabei sind alle elastischen Federelemente einsetzbar, die dem Fachmann grundsätzlich geläufig sind.

Es ist günstig, wenn ein Anschlag vorgesehen ist, der die Annäherung der beiden Anlageelemente unter der Wirkung des elastischen Spannelementes begrenzt, so daß die beiden Anlageelemente durch das elastische Spannelement nicht vollständig zusammengeschoben werden. Dies ist von Bedeutung, da der Operateur auf diese Weise die Möglichkeit hat, die beiden Anlageelemente zum Anlegen an dem Knochenteil elastisch auseinanderzuschieben und das Knochenteil zwischen die Anlageelemente zu positionieren.

Beispielsweise kann das elastische Spannelement mindestens ein elastisch dehnbares Zugglied umfassen, welches an beiden Anlageelementen angreift und diese elastisch gegeneinanderzieht.

Dabei kann das Zugglied in sich geschlossen sein und beide Anlageelemente außenseitig umschließen. Es ist auch möglich, daß das Zugglied zwischen beiden Anlageelementen angeordnet ist und mit jeweils einem Ende an je einem Anlageelement festgelegt ist.

Derartige Zugglieder können durch Gummiringe, Gummifäden oder ähnliche Strukturen gebildet sein, aber auch durch Schraubenfedern und andere elastische dehnbare Zugglieder.

Bei einer weiteren bevorzugten, jedoch nicht erfindungsgmäßen Ausgestaltung liegt das elastische Spannelement an der Oberseite des zweiten Anlageelementes an und stützt sich an der Spanneinrichtung ab. In diesem Falle wird das elastische Spannelement elastisch komprimiert oder verformt und übt dadurch Spannkräfte auf das zweite Anlageelement aus.

So kann beispielsweise das elastische Spannelement durch mindestens ein federndes Teil des zweiten Anlageelementes gebildet werden, das sich an der Spanneinrichtung abstützt. Beispielsweise kann es sich dabei um eine oder mehrere aus dem zweiten Anlageelement herausgearbeitete Federzungen handeln.

Bei einer bevorzugten, jedoch nicht erfindungsgmäßen Ausgestaltung weist die Spanneinrichtung mindestens einen am ersten Anlageelement gehaltenen und das zweite Anlageelement durchsetzenden Stab auf und an diesem Stab ist ein Abstützglied für das elastische Spannelement gehalten.

Dabei kann es sich beispielsweise um eine auf dem Stab nur in Richtung auf die beiden Anlageelemente verschiebbare Spannscheibe handeln, die im angelegten Zustand der Fixiereinrichtung die beiden Anlageelemente dauerhaft gegeneinander spannt.

Das Abstützglied kann bei einem anderen, jedoch nicht erfindungsgmäßen Ausführungsbeispiel auch am freien Ende des Stabes angeordnet sein, beispielsweise kann es auf das Ende des Stabes lösbar aufgesteckt oder aufgeschraubt sein.

Insbesondere kann das elastische Spannelement als eine den Stab umgebende Schraubenfeder ausgebildet sein.

Es ist günstig, wenn die Anlageelemente in Richtung auf das jeweils andere Anlageelement weisende, spitze Fixiervorsprünge tragen, durch die sie in die Knochensubstanz eintreten und dadurch an den Knochenteilen gehalten werden.

Die Fixiervorsprünge können gegenüber der Spanneinrichtung der beiden Anlageelemente geneigt sein. Dabei ist es günstig, wenn die Fixiervorsprünge aufeinander zu geneigt sind, wenn also die Abstände der Fixiervorsprünge an den Anlageelementen größer ist als die Abstände an den freien Enden der Fixiervorsprünge. Dadurch wird das seitliche Einschieben der Knochenteile zwischen die Anlageelemente erleichtert.

Die nachfolgende Beschreibung bevorzugter Ausführungsformen der Erfindung dient im Zusammenhang mit der Zeichnung der näheren Erläuterung. Es zeigen:
- Figur 1:: eine perspektivische Ansicht eines ersten bevorzugten Ausführungsbeispiels einer Fixiereinrichtung mit einer zwischen die Knochenteile und das zweite Anlageelement eingeschobenen Bügelfeder;
- Figur 2:: eine Ansicht ähnlich Figur 1 mit herausgezogener Bügelfeder und mit einer schematisch dargestellten Vorrichtung zum Spannen der beiden Anlageelemente gegeneinander;
- Figur 3:: eine Ansicht ähnlich Figur 1 bei einem weiteren Ausführungsbeispiel einer Fixiereinrichtung mit einem an Spannstäben gehaltenen elastischen Spannelement;
- Figur 4:: eine Ansicht ähnlich Figur 3 nach Entfernung des elastischen Spannelementes und vor dem Aufsetzen einer Brücke zur Verbindung der beiden Spannstäbe;
- Figur 5:: eine Ansicht ähnlich Figur 1 bei einem weiteren, jedoch nicht erfindungsgemäßem Ausführungsbeispiel einer Fixiereinrichtung mit einem die beiden Anlageelemente umgebenden, elastisch dehnbaren Spannelement;
- Figur 6:: eine Ansicht ähnlich Figur 5 bei einem weiteren, jedoch nicht erfindungsgemäßem Ausführungsbeispiel mit zwischen den Anlageelementen angeordneten, diese elastisch gegeneinander spannenden elastisch dehnbaren Spannelementen;
- Figur 7:: eine Ansicht ähnlich Figur 5 mit einer einen Spannstab umgebenden Schraubenfeder zum elastischen Gegeneinanderspannen der beiden Anlageelemente;
- Figur 8:: eine Ansicht ähnlich Figur 7 mit einer einen Spannstab umgebenden Schraubenfeder und einer Abstützung am freien Ende des Spannstabes und
- Figur 9:: eine Ansicht ähnlich Figur 7 mit Federzungen in dem zweiten Anlageelement zum elastischen Gegeneinanderspannen der beiden Anlageelemente.

Die in der Zeichnung dargestellte Fixiereinrichtung 1 dient der Verbindung von zwei nebeneinander liegenden Knochenteilen 2, 3, die in der Zeichnung nur ganz schematisch dargestellt sind. Es kann sich dabei handeln um zwei plattenförmige Teile des Schädelknochens, um zwei voneinander durch einen Sägeschnitt getrennte Teile des Brustbeines oder um ähnliche Knochenteile, die seitlich nebeneinanderliegend relativ zueinander fixiert werden sollen, wobei zwischen den beiden Knochenteilen 2, 3 ein schmaler Zwischenraum 4 verbleibt.

Die Fixiereinrichtung 1 umfaßt eine rechteckige, erste Anlageplatte 5 sowie eine zweite, ebenfalls rechteckige Anlageplatte 6. Beide Anlageplatten tragen an ihren Längskanten zur jeweils anderen Anlageplatte hin weisende, im wesentlichen quer von den ebenen Anlageplatten abstehende, zahnförmige Vorsprünge 7, die bei gegen die Knochenteile 2, 3 gespannten Anlageplatten 5, 6 in das Knochenmaterial eindringen und die Anlageplatten 5, 6 gegen eine seitliche Verschiebung relativ zu den Knochenteilen 2, 3 sichern.

Die erste Anlageplatte 5 wird zur Fixierung der Knochenteile 2, 3 an die Unterseite der Knochenteile 2, 3 angelegt, und zwar derart, daß sie den Zwischenraum 4 überbrückt, und in gleicher Weise wird die zweite Anlageplatte 6 an die Oberseite der Knochenteile 2, 3 angelegt. Wenn die beiden Anlageplatten 5, 6 in dieser Lage kräftig gegeneinander gespannt werden, sind die beiden Knochenteile 2, 3 gegeneinander fixiert.

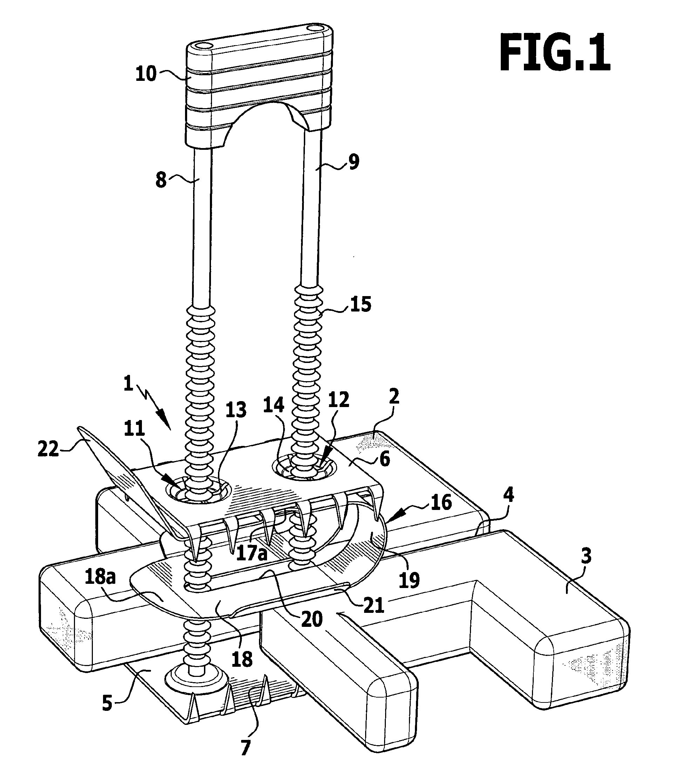

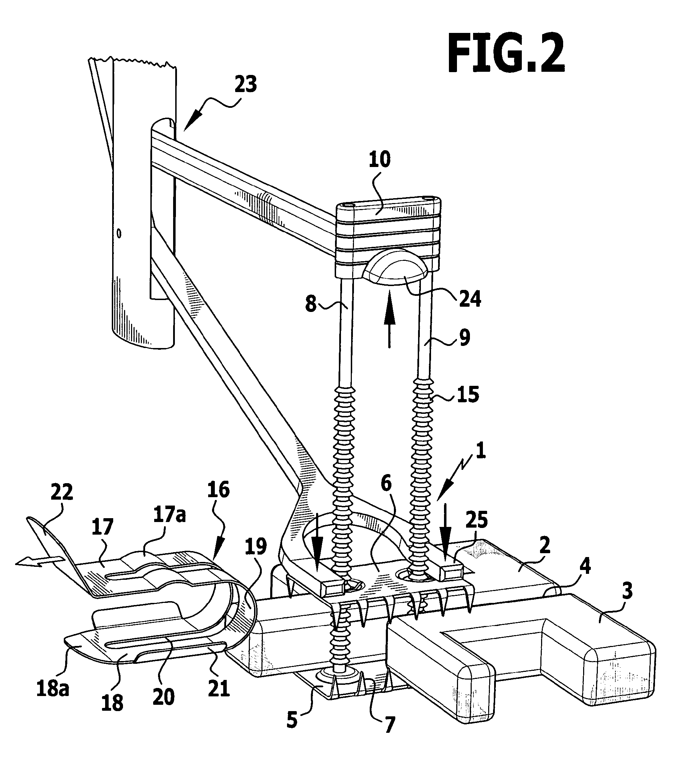

Die erste Anlageplatte 5 trägt zwei parallel nebeneinander angeordnete und senkrecht zur Anlageplatte verlaufende Spannstäbe 8, 9, die auf der Längsmittellinie der ersten Anlageplatte 5 angeordnet sind und die bei angelegter Fixiereinrichtung 1 durch den Zwischenraum 4 nach außen hindurchragen, dabei wird vorzugsweise die Breite des Zwischenraumes 4 durch den Durchmesser der Spannstäbe 8, 9 bestimmt. Bei dem in den Figuren 1 und 2 dargestellten Ausführungsbeispiel sind die beiden Spannstäbe 8, 9 an ihrem freien Ende durch eine Brücke 10 miteinander verbunden, die axial unverschieblich auf den Spannstäben 8, 9 gehalten ist, beispielsweise durch eine Verquetschung.

Die Spannstäbe 8, 9 durchsetzen die zweite Anlageplatte 6, die dazu zwei Durchbrechungen 11, 12 aufweist. Die Ränder dieser Durchbrechungen 11, 12 sind durch radiale Einschnitte 13 in elastische Zungen 14 unterteilt. Diese Zungen 14 biegen sich elastisch um, wenn die zweite Anlageplatte 6 in Richtung auf die erste Anlageplatte 5 verschoben wird, dabei gleiten diese Zungen 14 an Umfangsrippen 15 entlang, die an den beiden Spannstäben 8, 9 angeordnet sind. Es ist dadurch möglich, die zweite Anlageplatte 6 in Richtung auf die erste Anlageplatte 5 zu verschieben, nicht jedoch in umgekehrter Richtung, da die Zungen 14 nach dem elastischen Umbiegen eine Rückbewegung verhindern.

Zum Anlegen der Fixiereinrichtung der Figuren 1 und 2 an ein Knochenteil 2, 3 wird die zweite Anlageplatte 6 zunächst nur wenig in Richtung auf die erste Anlageplatte 5 verschoben, so daß der Abstand der beiden Anlageplatten 5, 6 deutlich größer ist als die Dicke der zu fixierenden Knochenteile 2, 3. Die Fixiereinrichtung 1 wird an einem der beiden Knochenteile 2 oder 3 zunächst so positioniert, daß die erste Anlageplatte 5 die gewünschte Position erhält, und dann wird in den Zwischenraum zwischen die Oberseite dieses Knochenteils und die Unterseite der zweiten Anlageplatte 6 eine Bügelfeder 16 eingeschoben. Diese Bügelfeder 16 besteht aus einem elastisch federnden Bandmaterial und ist U-förmig gebogen, so daß sie zwei parallele Arme 17, 18 und einen diese beiden Arme 17, 18 verbindenden, im wesentlichen kreisförmig gebogenen Steg 19 aufweist. Der untere Arm ist an seinem freien Ende in Form einer Aufgleitfläche 18a nach unten umgebogen.

Längs der Bügelfeder 16 weist diese einen mittigen Schlitz 20 auf, der sich durch den gesamten Steg 19 bis in beide Arme 17, 18 hinein erstreckt und dessen Breite mindestens so groß ist wie der Durchmesser der Spannstäbe 8, 9. Es ist dadurch möglich, die Bügelfeder 16 mit dem Steg 19 voraus zwischen die zweite Anlageplatte 6 und die Oberseite des Knochenteils einzuschieben, dabei treten die beiden Spannstäbe 8, 9 in den Schlitz 20 ein, der gleichzeitig als Führung für die Bügelfeder 16 dient. Die Einschubtiefe wird durch das Ende des Schlitzes 20 definiert, bei vollständig eingeschobener Bügelfeder 16 schlägt nämlich einer der beiden Spannstäbe 8, 9 an diesem Ende des Schlitzes 20 an (Figur 1).

Bei geeignetem Abstand der zweiten Anlageplatte 6 von der ersten Anlageplatte 5 spannt diese elastisch zusammendrückbare Bügelfeder 16 die erste Anlageplatte 5 gegen die Unterseite des Knochenteils, d.h. das Knochenteil wird zwischen dem unteren Arm 18 der Bügelfeder 16 und der ersten Anlageplatte 5 eingespannt (Figur 1). Der Abstand der beiden Anlageplatten 5, 6 kann dabei durch Verschiebung der zweiten Anlageplatte 6 in Richtung auf die erste Anlageplatte 5 in geeigneter Weise eingestellt werden, so daß gegebenenfalls auch die Federkraft dadurch entsprechend eingestellt werden kann, mit der die Fixiereinrichtung 1 am Knochenteil gehalten ist.

Nach der Fixierung der Fixiereinrichtung 1 an einem der beiden Knochenteile 2 kann das andere Knochenteil 3 nunmehr seitlich in den Zwischenraum zwischen dem unteren Arm 18 der Bügelfeder 16 und der ersten Anlageplatte 5 eingeschoben werden, bis das Knochenteil an den Spannstäben 8, 9 anschlägt. Dabei wird der untere Arm 18 der Bügelfeder 16 federn nach oben gedrückt, so daß das Knochenteil auch an den vorstehenden Vorsprüngen 7 vorbei gleiten kann.

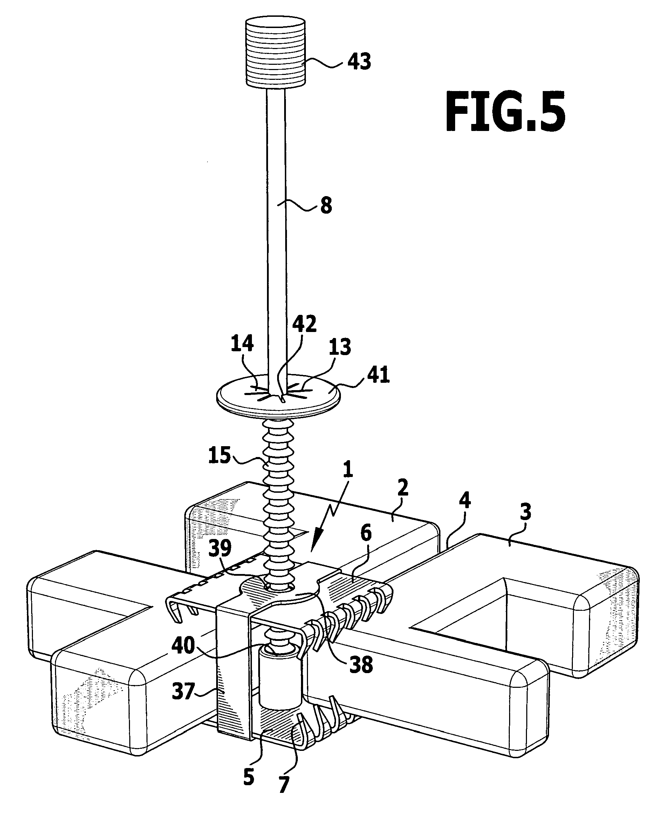

Diese Vorsprünge 7 können senkrecht von den beiden Anlageplatten 5, 6 abstehen, es ist aber auch möglich, diese Vorsprünge 7 geringfügig nach innen zu neigen, wie dies insbesondere aus den Figuren 5 bis 9 ersichtlich ist. Dadurch wird das Einführen der Knochenteile zwischen die erste Anlageplatte 5 und die Bügelfeder 16 erleichtert.

In ähnlicher Weise kann an den Seitenkanten des unteren Armes 18 der Bügelfeder 16 jeweils eine schräge Einlauffläche 21 vorgesehen werden, die beim Gegeneinanderschieben der Knochenteile 2, 3 an den Knochenteilen entlanggleitet und dadurch den unteren Arm 18 federnd anhebt (Figuren 1 und 2).

Die Fixiereinrichtung 1 wird durch die Bügelfeder 16 so gegen die Knochenteile 2, 3 gedrückt, daß die erste Anlageplatte 5 mit ihren Vorsprüngen 7 in die Knochenteile 2, 3 eingreift, und dadurch ergibt sich eine Fixierung der Fixiereinrichtung an den Knochenteilen 2, 3. Es ist daher möglich, nach dieser Fixierung die Bügelfeder 16 wieder zu entfernen, dies erfolgt einfach durch seitliches Herausziehen. Zu diesem Zweck trägt die Bügelfeder 16 ein Griffstück 22 in Form eines schräg nach oben abgebogenen Lappens, an dem die Bügelfeder 16 von Hand oder mittels eines Instrumentes ergriffen und dann herausgezogen werden kann. Obwohl damit die elastische Spannkraft entfällt, bleibt die Fixiereinrichtung in dieser Position, da sie durch die in das Knochenmaterial eingegrabenen Vorsprünge 7 in dieser Position gehalten wird. Nach Entfernung der Bügelfeder 16 ist es möglich, mit einem in Figur 2 nur sehr schematisch dargestellten Spanninstrument 23 die beiden Anlageplatten 5 und 6 gegeneinander zu spannen. Dazu legt sich das Spanninstrument 23 mit einem oberen Spannwerkzeug 24 an der Brücke 10 an und mit einem unteren Spannwerkzeug 25 an der Oberseite der zweiten Anlageplatte 6. Beim Spreizen der beiden Spannwerkzeuge 24 und 25 wird dadurch die zweite Anlageplatte 6 in Richtung auf die erste Anlageplatte 5 längs der beiden Spannstäbe 8, 9 verschoben, und dies führt zu einer endgültigen Fixierung der beiden Anlageplatten 5, 6 an den Knochenteilen 2, 3 (Figur 2). Danach können die überstehenden Teile der Spannstäbe 8, 9 entfernt werden, beispielsweise mit Hilfe eines Schneidwerkzeuges, und die Fixiereinrichtung 1 verbleibt im Körper. Die entfernten oberen Teile der Spannstäbe 8, 9 mit der Brücke 10 und die Bügelfeder 16 dagegen werden aus dem Operationsbereich entfernt.

Der obere Arm 17 der Bügelfeder 16 ist in seinem mittleren Bereich zwischen dem Steg 19 und seinem Ende nach oben aufgewölbt (Figur 2), diese Aufwölbung 17a greift bei vollständig eingeschobener Bügelfeder 16 zwischen die beiden Durchbrüche 11, 12 ein, die geringfügig nach unten über die zweite Anlageplatte 6 hervorstehen. Durch dieses Eingreifen der Aufwölbung 17a zwischen die beiden nach unten überstehenden Durchbrechungen 11, 12 ergibt sich eine Festlegung der Bügelfeder 16 gegen eine Verschiebung in Richtung des Schlitzes 20, d.h. die Bügelfeder 16 wird im eingeschobenen Zustand festgelegt. Diese Festlegung kann jedoch dadurch gelöst werden, daß kräftig an dem Griffstück 22 gezogen wird, dadurch gleitet die Aufwölbung 17a an der nach unten vorstehenden Durchbrechung 11 auf und biegt den oberen Arm 17 elastisch nach unten, so daß die Bügelfeder 16 vollständig aus dem Zwischenraum zwischen dem Knochenteil und der oberen Anlageplatte 6 herausgezogen werden kann.

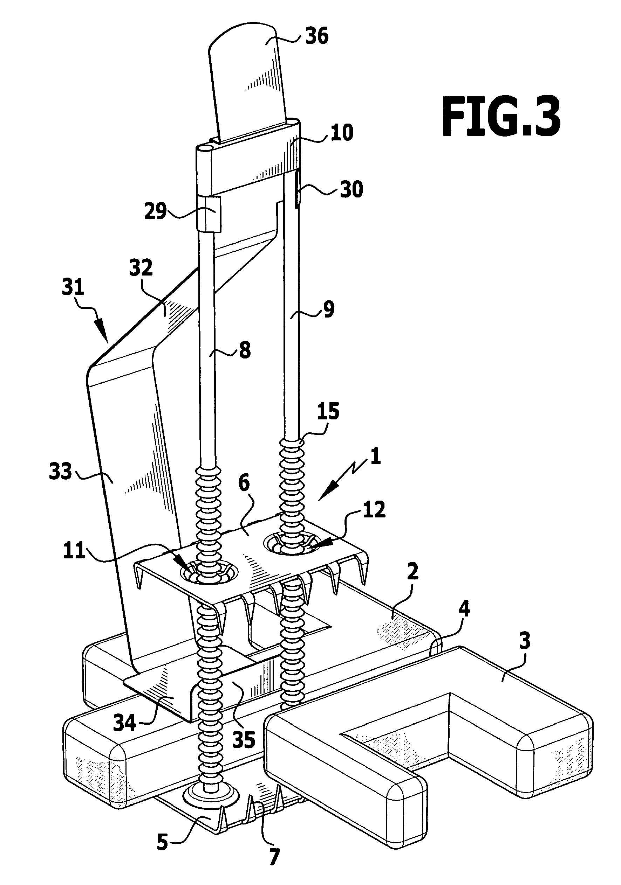

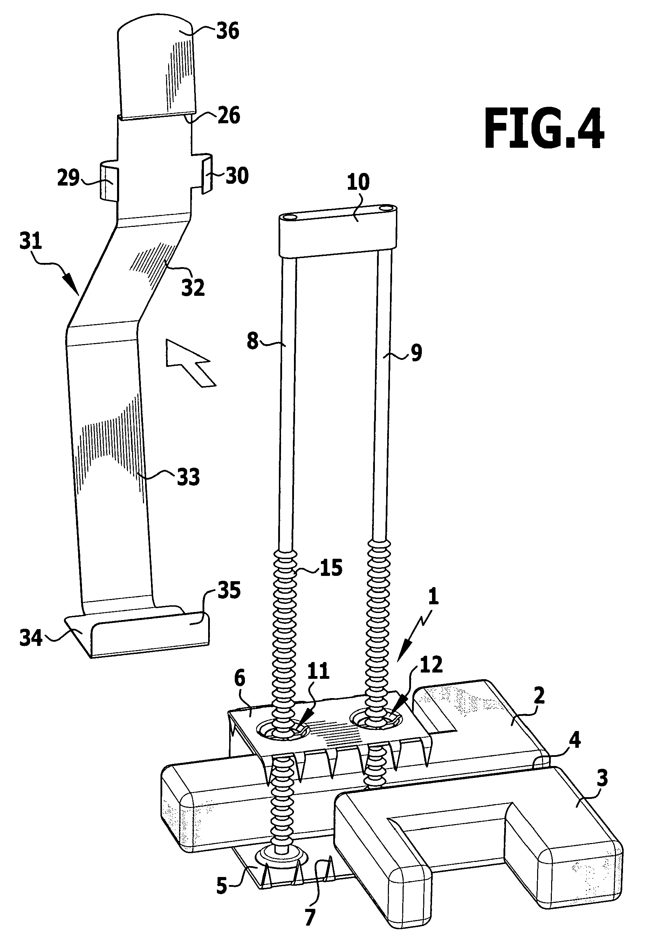

Bei dem in den Figuren 3 und 4 dargestellten Ausführungsbeispiel ist ein ähnlicher Aufbau gewählt, einander entsprechende Teile tragen daher dieselben Bezugszeichen.

An den beiden Stäben 8, 9 der Fixiereinrichtung 1, die an ihrem freien Ende über eine Brücke 10 miteinander verbunden sind, ist lösbar ein Federarm 31 gehalten, und zwar mit Hilfe von zwei Klemmgliedern 29 und 30, die die Spannstäbe 8, 9 teilweise umgreifen. Der Federarm besteht aus einem bandförmigen, elastisch verbiegbaren Material und weist eine Stufe 26 auf, die auf der Oberseite der Brücke 10 aufliegt, wenn der Federarm 31 mit der Fixiereinrichtung 1 verbunden ist. Der Federarm 31 weist zwei geradlinige, winklig aneinander anschließende Abschnitte 32 und 33 auf, und am unteren Ende des Federarmes 31 geht dieser über in eine parallel zu den beiden Anlageplatten 5, 6 verlaufende Stützplatte 34, die sich zwischen den beiden Anlageplatten 5 und 6 befindet und an ihrer dem Federarm 31 gegenüberliegenden Längskante einen rechtwinklig nach oben abstehenden Randbereich 35 aufweist.

Aufgrund der elastischen Eigenschaften des Federarmes 31 kann die Stützplatte 34 gegen die Federkraft des Federarmes 31 von der ersten Anlageplatte 5 entfernt werden, so daß die Fixiereinrichtung 1 so an ein Knochenteil angelegt werden kann, daß dieses zwischen der Stützplatte 34 und der ersten Anlageplatte 5 eingespannt wird. Der Federarm 31 drückt dabei die Stützplatte 34 federnd gegen die Oberseite des Knochenteils und spannt dabei die erste Anlageplatte 5 gegen die Unterseite des Knochenteils, wobei die zahnförmigen Vorsprünge 7 zumindest teilweise in das Knochenmaterial eindringen können. Auf diese Weise ist eine Festlegung der Fixiereinrichtung 1 an dem Knochenteil möglich, wobei die zweite Anlageplatte 6 noch im Abstand oberhalb der Knochenteile 2, 3 verbleibt (Figur 3).

In ähnlicher Weise wie bei dem Ausführungsbeispiel der Figuren 1 und 2 kann dann das zweite Knochenteil seitlich zwischen die Stützplatte 34 und die erste Anlageplatte 5 eingeschoben werden.

Bevor die beiden Anlageplatten 5, 6 endgültig fest gegeneinander gespannt werden, wird der Federarm 31 entfernt. Zu diesem Zweck werden die Klemmelemente 29, 30 aufgebogen. Der Lösevorgang wird unterstützt durch eine nach oben vorstehenden, lappenförmige Verlängerung 36, an welcher der Federarm 31 ergriffen werden kann.

Nach der Entfernung des Federarms 31 können die Anlageplatten 5, 6 in ähnlicher Weise wie bei dem Ausführungsbeispiel der Figur 2 endgültig gegeneinander gespannt werden.

Bei den Ausführungsbeispielen der Figuren 1 bis 4 wird die erste Anlageplatte 5 mittels einer Spannfläche gegen ein Knochenteil gespannt, im Ausführungsbeispiel der Figuren 1 und 2 bildet der untere Arm 18 die Spannfläche, im Ausführungsbeispiel der Figuren 3 und 4 die Stützplatte 34.

Im Gegensatz dazu sind in den Figuren 5 bis 9 nicht erfindungsgemäße Ausgestaltungen dargestellt, bei denen eine solche Spannfläche durch die zweite Anlageplatte 6 selbst gebildet wird. In diesen Fällen wird dafür Sorge getragen, daß die beiden Anlageplatten 5, 6 elastisch gegeneinander gespannt werden und dadurch eine Festlegung der Fixiereinrichtung 1 an einem Knochenteil ermöglichen.

Die in den Figuren 5 bis 9 dargestellten Fixiereinrichtungen 1 sind ähnlich aufgebaut wie die der Figuren 1 bis 4, einander entsprechende Teile tragen daher dieselben Bezugszeichen. Im Gegensatz zu den Ausführungsbeispielen der Figuren 1 bis 4 ist bei den Ausführungsbeispielen der Figuren 5 bis 9 nur ein einziger Spannstab 8 vorgesehen, es wäre aber ohne weiteres möglich, auch diese Ausführungsbeispiele in ähnlicher Weise wie bei den Ausführungsbeispielen der Figuren 1 bis 4 dargestellt mit zwei Spannstäben zu versehen.

Bei dem Ausführungsbeispiel der Figur 5 umgibt die beiden Anlageplatten 5, 6 ein außenseitig an diesen vorbei geführtes, in sich geschlossenes elastisches Band 37, im einfachsten Fall ein Gummiring oder ein vergleichbares elastisches Bauteil aus einem körperverträglichen Material, beispielsweise aus Silikon. Zur Festlegung dieses Bandes 37 weist dieses eine Verbreiterung 38 mit einer Öffnung 39 auf, diese Öffnung 39 liegt unmittelbar über der Durchbrechung 11 der zweiten Anlageplatte 6, so daß der Spannstab 8 auch diese Öffnung 39 auf, diese Öffnung 39 liegt unmittelbar über der Durchbrechung 11 der zweiten Anlageplatte 6, so daß der Spannstab 8 auch diese Öffnung 39 durchsetzt.

Durch das Band 37 werden die beiden Anlageplatten 5, 6 elastisch gegeneinander gespannt, eine vollständige Annäherung unter der Einwirkung des elastischen Bandes 37 wird dabei durch eine stufenförmige Verbreiterung 40 des Spannstabes 8 verhindert, diese stufenförmige Verbreiterung 40 schließt sich unmittelbar an die erste Anlageplatte 5 an und bildet einen Anschlag für die zweite Anlageplatte 6.

Bei dem Ausführungsbeispiel der Figur 5 ist die zweite Anlageplatte 6 auf dem Spannstab 8 in beiden Richtungen frei verschieblich, es fehlen also die radialen Einschnitte 13 und die Zunge 14, die bei den Ausführungsbeispielen der Figuren 1 bis 4 vorgesehen sind. Dadurch ist es möglich, den Abstand der zwei Anlageplatten 5, 6 gegen die Wirkung des elastischen Bandes 37 zu vergrößern und an die Dicke der Knochenteile anzupassen.

Zum endgültigen Gegeneinanderspannen der beiden Anlageplatten 5, 6 ist auf dem Spannstab 8 eine Arretierscheibe 41 gelagert mit einer zentralen Öffnung 42, durch die der Spannstab 8 hindurchgreift. Diese Öffnung 42 ist ähnlich ausgebildet wie die Durchbrechung 11 bei den Ausführungsbeispielen der Figuren 1 bis 4, es sind nämlich radiale Einschnitte 13 vorgesehen, die die Arretierscheibe 41 im Randbereich der Öffnung 42 in eine Anzahl von nebeneinanderliegenden federnden Zungen 14 unterteilen. Auf diese Weise ist die Arretierscheibe 41 ebenso wie die zweite Anlageplatte 6 bei den Ausführungsbeispielen der Figuren 1 bis 4 von oben nach unten verschiebbar, wegen der Umfangsrippen 15 jedoch nicht von unten nach oben.

Die Verschiebung der Arretierscheibe 41 erfolgt mit einem Spanninstrument, das ähnlich ausgebildet ist wie das Spanninstrument 23 beim Ausführungsbeispiel der Figur 2, in diesem Falle stützt sich das obere Werkzeug dann an einer kopfförmigen Verdickung 43 des Spannstabes 8 ab. Das elastische Band 37 kann nach dem Festlegen der Fixiereinrichtung 1 an den Knochenteilen 2, 3 entfernt werden, bevor die Arretierscheibe 41 in die endgültige Spannstellung verschoben wird, diese Entfernung kann beispielsweise einfach dadurch erfolgen, daß das elastische Band aufgeschnitten und seitlich weggezogen wird.

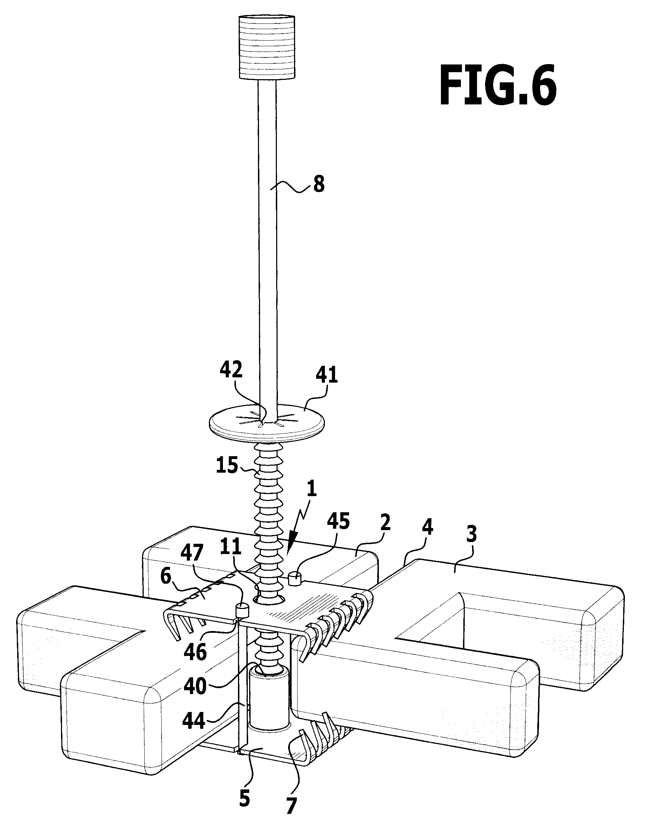

Bei dem Ausführungsbeispiel der Figur 6 ist eine ähnliche Anordnung gewählt wie bei dem Ausführungsbeispiel der Figur 5, einander entsprechende Teile tragen daher dieselben Bezugszeichen.

In diesem Falle werden die beiden Anlageplatten 5, 6 nicht durch ein sie umgebendes elastisches Band gegeneinander gespannt, sondern durch zwei elastische dehnbare Spannelemente 44, 45, die mit ihren Enden jeweils an beiden Anlageplatten 5, 6 festgelegt sind und im Zwischenraum zwischen den beiden Anlageplatten 5, 6 angeordnet sind. Die Anordnung ist dabei so gewählt, daß die beiden Spannelemente 44, 45 ebenso im Zwischenraum 4 der beiden Knochenteile 2, 3 liegen.

Bei den Spannelementen 44, 45 kann es sich um Zugfedern handeln oder um gummielastische Bänder.

Auch diese Spannelemente 44, 45 können entfernt werden, in diesem Falle ist die Entfernung auch noch möglich, wenn die Anlageplatten 5, 6 durch die Arretierscheibe 41 vollständig gegeneinander gespannt sind.

Die Spannelemente 44, 45 können beispielsweise durch seitlich offene Schlitze 46 in den Anlageplatten 5, 6 in diese eingesetzt werden und werden durch eine kopfförmige Verdickung 47 in axialer Richtung fixiert. Dadurch ist es möglich, die Spannelemente 44, 45 seitlich aus diesen Schlitzen 46 herauszuziehen und dadurch von den Anlageplatten 5, 6 zu lösen, wenn die Spannelemente 44, 45 entfernt werden sollen.

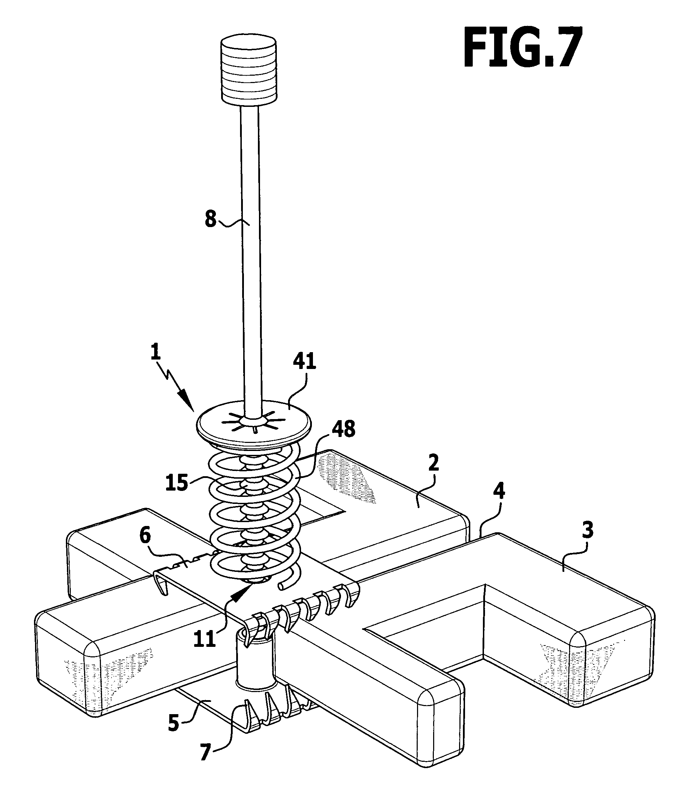

Bei dem Ausführungsbeispiel der Figur 7 ist ein ähnlicher Aufbau gewählt wie bei dem Ausführungsbeispiel der Figur 5, einander entsprechende Teile tragen daher dieselben Bezugszeichen.

In diesem Falle werden die beiden Anlageplatten 5, 6 nicht durch ein elastisches Band gegeneinander gespannt, sondern durch eine Schraubenfeder 48, die den Spannstab 8 konzentrisch umgibt und die sich einerseits an der Oberseite der zweiten Anlageplatte 6 und andererseits an der Unterseite der Arretierscheibe 41 abstützt und dadurch die zweite Anlageplatte 6 elastisch gegen die erste Anlageplatte 5 verschiebt.

Bei diesem Ausführungsbeispiel wird bei dem endgültigen Zusammenspannen der Anlageplatten 5, 6 diese Schraubenfeder 48 vollständig komprimiert, es wäre aber auch möglich, diese Schraubenfeder 48 zu entfernen, beispielsweise dadurch, daß die aus dünnem Federdraht gewickelte Schraubenfeder 48 seitlich abgezogen wird, so daß sich der Federdraht streckt.

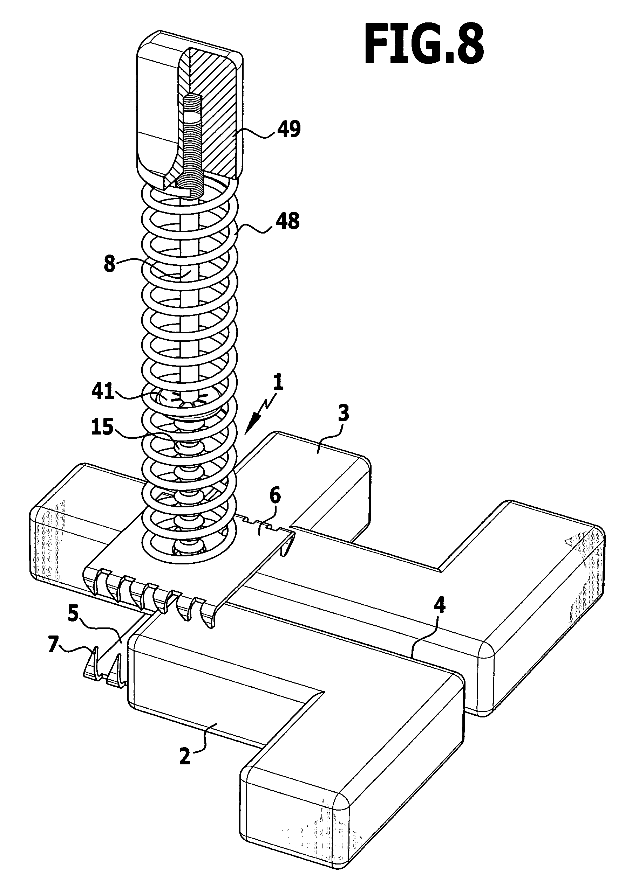

Eine ähnliche Ausgestaltung ist verwirklicht bei dem Ausführungsbeispiel der Figur 8.

Im Unterschied zum Ausführungsbeispiel der Figur 7 umgibt die Schraubenfeder 48 bei diesem Ausführungsbeispiel die Arretierscheibe 41 im Abstand und stützt sich an einem Anschlag 49 ab, der auf das freie Ende des Spannstabes 8 aufgesetzt ist. Der Anschlag 49 kann beispielsweise im Klemmsitz auf dem Spannstab 8 gehalten werden oder durch ein Gewinde, auf das der Anschlag 49 aufgeschraubt ist, oder durch eine Bajonettverriegelung. Dadurch ist es möglich, den Anschlag 49 nach der Positionierung der Fixiereinrichtung 1 an den Knochenteilen zu entfernen und die Schraubenfeder 48 nach oben abzuziehen, so daß der Spannstab 8 und die Arretierscheibe 41 in der aus den Figuren 5 bis 7 ersichtlichen Weise freigegeben werden. Der Anschlag 49 kann wieder auf den Spannstab 8 aufgesetzt werden und wirkt dann in ähnlicher Weise wie die kopfförmige Verdickung 43 als Anschlag für ein Spanninstrument.

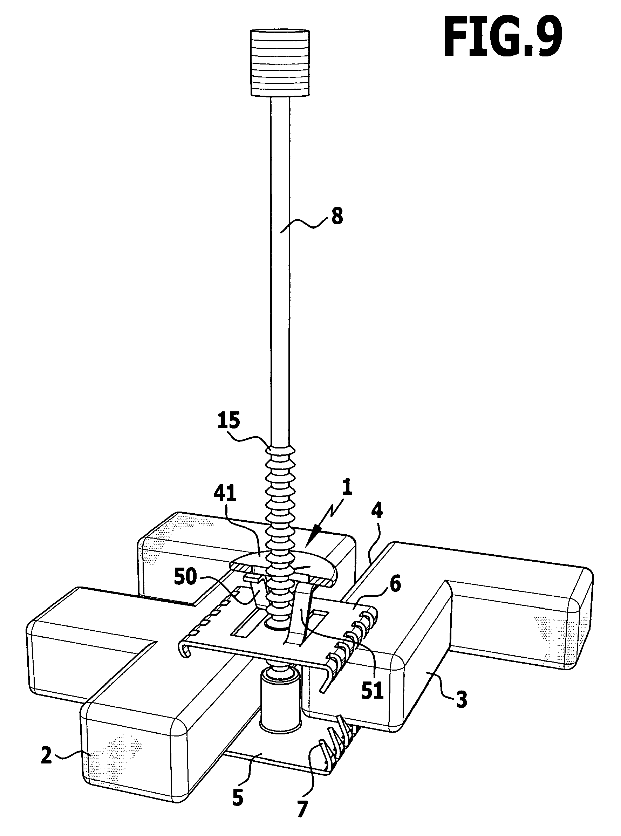

Bei dem Ausführungsbeispiel der Figur 9, das ähnlich aufgebaut ist wie das der Figur 5 und bei dem dieselben Teile wieder dieselben Bezugszeichen tragen, sind aus der zweiten Anlageplatte 6 durch U-förmige Schnitte zungenförmige Bereiche ausgestanzt, die nach oben abgebogen sind und die Federzungen 50, 51 ausbilden. Diese stützen sich an der Arretierscheibe 41 ab und spannen dadurch die Anlageplatten 5, 6 federnd gegeneinander. Der Abstand kann dabei gegen die Federkraft dieser Federzungen 50, 51 vergrößert werden, so daß die Fixiereinrichtung 1 seitlich auf die Knochenteile aufgeschoben bzw. die Knochenteile zwischen die Anlageplatten 5, 6 eingeschoben werden können. Zum endgültigen Spannen der Anlageplatten 5, 6 wird die Arretierscheibe 41 nach unten verschoben und biegt dabei gleichzeitig auch die Federzungen 50, 51 soweit nach unten, daß die Arretierscheibe 41 auf der Oberseite der zweiten Anlageplatte 6 zur Anlage kommt.

## Patentansprüche

1. Chirurgische Fixiereinrichtung (1) zur gegenseitigen Fixierung von zwei nebeneinanderliegenden Knochenteilen (2, 3) mit einem ersten Anlageelement (5) zur Anlage an der Unterseite der beiden Knochenteile (2, 3) und mit einem zweiten Anlageelement (6) zur Anlage an der Oberseite der beiden Knochenteile (2, 3) und mit einer Spanneinrichtung (8, 9), die die beiden Anlageelemente (5, 6) durch einen Zwischenraum zwischen den Knochenteilen (2, 3) hindurch miteinander verbindet und die die beiden Anlageelemente (5, 6) dauerhaft gegeneinander gespannt fixiert, wenn sie an der Unterseite bzw. der Oberseite der Knochenteile (2, 3) anliegen, wobei die Fixiereinrichtung (1) mindestens ein elastisches Spannelement (16; 31) umfaßt, welches eine an die Oberseite der Knochenteile (2, 3) anlegbare Stützfläche (18; 34) gegen das erste Anlageelement (5) verschiebt, so daß die Stützfläche (18; 34) und das erste Anlageelement (5) gegen die Kraft des elastischen Spannelementes (16; 31) voneinander entfernbar sind, und wobei die Stützfläche (18; 34) Teil des elastischen Spannelements (16; 31) ist, **dadurch gekennzeichnet, daß** das elastische Spannelement (16; 31) mit der Fixiereinrichtung (1) lösbar verbunden ist.

2. Fixiereinrichtung nach Anspruch 1, **dadurch gekennzeichnet, daß** die Stützfläche (18; 34) vor dem Anlegen der Fixiereinrichtung (1) an die Knochenteile (2, 3) zwischen den beiden Anlageelementen (5, 6) angeordnet ist.

3. Fixiereinrichtung nach Anspruch 1 oder 2, **dadurch gekennzeichnet, daß** das elastische Spannelement (31) an der Spanneinrichtung (8, 9) gehalten ist.

4. Fixiereinrichtung nach Anspruch 3, **dadurch gekennzeichnet, daß** die Spanneinrichtung mindestens einen am ersten Anlageelement (5) gehaltenen und das zweite Anlageelement (6) durchsetzenden Stab (8, 9) aufweist und daß das elastische Spannelement (31) an diesem Stab (8, 9) gehalten ist.

5. Fixiereinrichtung nach Anspruch 4, **dadurch gekennzeichnet, daß** das elastische Spannelement (31) am freien Ende dieses Stabes (8, 9) gehalten ist.

6. Fixiereinrichtung nach Anspruch 4 oder 5, **dadurch gekennzeichnet, daß** die Spanneinrichtung zwei nebeneinander angeordnete Stäbe (8, 9) aufweist und daß das elastische Spannelement (31) einen die beiden Stäbe (8, 9) verbindenden Steg (26) aufweist.

7. Fixiereinrichtung nach einem der Ansprüche 4 bis 6, **dadurch gekennzeichnet, daß** das Spannelement (31) mindestens ein Klemmelement (29, 30) trägt, welches das Spannelement (31) an dem Stab oder den Stäben (8, 9) lösbar festlegt.

8. Fixiereinrichtung nach einem der Ansprüche 4 bis 7, **dadurch gekennzeichnet, daß** das Spannelement einen die Stützfläche (34) und die Verbindungsstelle mit der Spanneinrichtung (8, 9) verbindenden, seitlich an dem zweiten Anlageelement (6) vorbei geführten Federarm (31) aufweist.

9. Fixiereinrichtung nach einem der Ansprüche 4 bis 8, **dadurch gekennzeichnet, daß** das Spannelement (31) ein die Spanneinrichtung (8, 9) überragendes Griffstück (36) trägt.

10. Fixiereinrichtung nach einem der voranstehenden Ansprüche, **dadurch gekennzeichnet, daß** sich das elastische Spannelement (16) an dem zweiten Anlageelement (6) abstützt.

11. Fixiereinrichtung nach Anspruch 10, **dadurch gekennzeichnet, daß** das elastische Spannelement (16) bei an die Knochenteile (2, 3) angelegter Fixiereinrichtung (1) seitlich zwischen die Oberseite der Knochenteile (2, 3) und die Unterseite des zweiten Anlageelementes (6) eingeschoben und seitlich wieder herausschiebbar ist.

12. Fixiereinrichtung nach einem der Ansprüche 10 oder 11, **dadurch gekennzeichnet, daß** das elastische Spannelement eine Bügelfeder (16) ist.

13. Fixiereinrichtung nach Anspruch 12, **dadurch gekennzeichnet, daß** die Bügelfeder (16) U-förmig ausgebildet ist mit einem die Stützfläche bildenden ersten Arm (18) und einem an dem zweiten Anlageelement (6) anlegbaren zweiten Arm (17) und mit einem die beiden Arme (17, 18) verbindenden, federnden Steg (19).

14. Fixiereinrichtung nach einem der Ansprüche 10 bis 13, **dadurch gekennzeichnet, daß** die Spanneinrichtung mindestens einen am ersten Anlageelement (5) gehaltenen und das zweite Anlageelement (6) durchsetzenden Stab (8, 9) aufweist und daß das Spannelement (16) auf beiden Seiten des Stabes oder der Stäbe (8, 9) angeordnet ist.

15. Fixiereinrichtung nach Anspruch 14, **dadurch gekennzeichnet, daß** das Spannelement (16) eine schlitzförmige Ausnehmung (20) aufweist, in die der Stab oder die Stäbe (8, 9) der Spanneinrichtung beim Einschieben des elastischen Spannelements (16) eintreten.

16. Fixiereinrichtung nach einem der Ansprüche 11 bis 15, **dadurch gekennzeichnet, daß** mindestens ein Arretierglied (17a) vorgesehen ist, welches das elastische Spannelement (16) im eingeschobenen Zustand gegen ein Herausschieben sichert, und daß das Arretierglied (17a) durch einen Grenzwert überschreitende Verschiebekräfte lösbar ist.

17. Fixiereinrichtung nach einem der Ansprüche 11 bis 16, **dadurch gekennzeichnet, daß** das elastische Spannelement (16) im eingeschobenen Zustand des elastischen Spannelementes (16) ein seitlich über das zweite Anlageelement (6) vorstehendes Griffstück (18a) trägt.

## Claims

1. Surgical fixing device (1) for the mutual fixing of two bone parts (2, 3) lying next to each other, having a first engaging element (5) for engaging the underside of the two bone parts (2, 3) and having a second engaging element (6) for engaging the upper side of the two bone parts (2, 3) and having a tensioning device (8, 9), which connects the two engaging elements (5, 6) to each other through an interspace between the bone parts (2, 3) and which permanently fixes the two engaging elements (5, 6) with respect to each other in a tensioned manner when they engage the underside and the upper side of the bone parts (2, 3), wherein the fixing device (1) comprises at least one elastic tensioning element (16; 31), which displaces a supporting surface (18; 34) which can be applied to the upper side of the bone parts (2, 3) toward the first engaging element (5), so that the supporting surface (18; 34) and the first engaging element (5) can be moved away from each other against the force of the elastic tensioning element (16; 31), and wherein the supporting surface (18; 34) is part of the elastic tensioning element (16; 31), **characterized in that** the elastic tensioning element (16; 31) is releasably connected to the fixing device (1).

2. Fixing device in accordance with Claim 1, **characterized in that** the supporting surface (18; 34) is disposed between the two engaging elements (5, 6) before the fixing device (1) is applied to the bone parts (2, 3).

3. Fixing device in accordance with Claim 1 or 2, **characterized in that** the elastic tensioning element (31) is held on the tensioning device (8, 9).

4. Fixing device in accordance with Claim 3, **characterized in that** the tensioning device has at least one rod (8, 9) that is held on the first engaging element (5) and passes through the second engaging element (6) and **in that** the elastic tensioning element (31) is held on this rod (8, 9).

5. Fixing device in accordance with Claim 4, **characterized in that** the elastic tensioning element (31) is held at the free end of this rod (8, 9).

6. Fixing device in accordance with Claim 4 or 5, **characterized in that** the tensioning device has two rods (8, 9) disposed next to each other and **in that** the elastic tensioning element (31) has a cross-piece (26) connecting the two rods (8, 9).

7. Fixing device in accordance with any one of Claims 4 to 6, **characterized in that** the tensioning element (31) carries at least one clamping element (29, 30), which releasably fixes the tensioning element (31) on the rod or the rods (8, 9).

8. Fixing device in accordance with any one of Claims 4 to 7, **characterized in that** the tensioning element has a spring arm (31) which connects the supporting surface (34) and the location where the tensioning element is connected to the tensioning device (8, 9) and is brought laterally past the second engaging element (6).

9. Fixing device in accordance with any one of Claims 4 to 8, **characterized in that** the tensioning element (31) carries a grip (36) extending beyond the tensioning device (8, 9).

10. Fixing device in accordance with any one of the preceding claims, **characterized in that** the elastic tensioning element (16) is supported on the second engaging element (6).

11. Fixing device in accordance with Claim 10, **characterized in that** the elastic tensioning element (16) is pushed laterally in between the upper side of the bone parts (2, 3) and the underside of the second engaging element (6), and can be pushed laterally out again, when the fixing device (1) is engaging the bone parts (2, 3).

12. Fixing device in accordance with Claim 10 or 11, **characterized in that** the elastic tensioning element is a bow spring (16).

13. Fixing device in accordance with Claim 12, **characterized in that** the bow spring (16) is formed in a U-shaped manner, with a first arm (18) that forms the supporting surface and a second arm (17) that can be applied to the second engaging element (6) and with a resilient cross-piece (19) that connects the two arms (17, 18).

14. Fixing device in accordance with any one of Claims 10 to 13, **characterized in that** the tensioning device has at least one rod (8, 9) that is held on the first engaging element (5) and passes through the second engaging element (6) and **in that** the tensioning element (16) is disposed on both sides of the rod or rods (8, 9).

15. Fixing device in accordance with Claim 14, **characterized in that** the tensioning element (16) has a slit-shaped cut-out (20), which is entered by the rod or rods (8, 9) of the tensioning device when the elastic tensioning element (16) is pushed in.

16. Fixing device in accordance with any one of Claims 11 to 15, **characterized in that** at least one arresting member (17a) is provided, which secures the elastic tensioning element (16) to prevent it from being pushed out in the pushed-in state, and **in that** the arresting member (17a) can be released by displacing forces exceeding a limit value.

17. Fixing device in accordance with any one of Claims 11 to 16, **characterized in that** the elastic tensioning element (16) carries a grip (18a) that extends laterally beyond the second engaging element (6) in the pushed-in state of the elastic tensioning element (16).

## Revendications

1. Dispositif de fixation chirurgical (1) pour la fixation réciproque de deux parties d'os (2, 3) placées côte à côte, comprenant un premier élément d'appui (5) destiné à venir en appui sur le côté inférieur des deux parties d'os (2, 3), et un deuxième élément d'appui (6) destiné à venir en appui sur le côté supérieur des deux parties d'os (2, 3), et comprenant également un dispositif de serrage (8, 9), qui relie mutuellement les deux éléments d'appui (5, 6) à travers un espace intermédiaire entre les parties d'os (2, 3), et fixe les deux éléments d'appui (5, 6) serrés de manière durable l'un contre l'autre lorsqu'ils s'appuient respectivement contre le côté inférieur et le côté supérieur des parties d'os (2, 3),
le dispositif de fixation (1) comportant au moins un élément de serrage élastique (16; 31), qui déplace, vers le premier élément d'appui (5), une surface de soutien (18; 34) pouvant être appliquée contre le côté supérieur des parties d'os (2, 3), de sorte que la surface de soutien (18; 34) et le premier élément d'appui (5) peuvent être éloignés l'un de l'autre à l'encontre de la force de l'élément de serrage élastique (16; 31),
et la surface de soutien (18; 34) faisant partie intégrante de l'élément de serrage élastique (16; 31), **caractérisé en ce que** l'élément de serrage élastique (16; 31) est relié de manière amovible au dispositif de fixation (1).

2. Dispositif de fixation selon la revendication 1, **caractérisé en ce que** la surface de soutien (18; 34) est agencée entre les deux éléments d'appui (5, 6) avant l'application du dispositif de fixation (1) sur les parties d'os (2, 3).

3. Dispositif de fixation selon la revendication 1 ou la revendication 2, **caractérisé en ce que** l'élément de serrage élastique (31) est maintenu sur le dispositif de serrage (8, 9).

4. Dispositif de fixation selon la revendication 3, **caractérisé en ce que** le dispositif de serrage comprend au moins une broche (8, 9) maintenue sur le premier élément d'appui (5) et traversant le deuxième élément d'appui (6), et **en ce que** l'élément de serrage élastique (31) est maintenu sur cette broche (8, 9).

5. Dispositif de fixation selon la revendication 4, **caractérisé en ce que** l'élément de serrage élastique (31) est maintenu à l'extrémité libre de cette broche (8, 9).

6. Dispositif de fixation selon la revendication 4 ou la revendication 5, **caractérisé en ce que** le dispositif de serrage comprend deux broches (8, 9) agencées côte à côte, et **en ce que** l'élément de serrage élastique (31) présente une branche (26) reliant les deux broches (8, 9).

7. Dispositif de fixation selon l'une des revendications 4 à 6, **caractérisé en ce que** l'élément de serrage (31) porte au moins un élément de blocage (29, 30), qui fixe de manière amovible l'élément de serrage (31) sur la broche ou les broches (8, 9).

8. Dispositif de fixation selon l'une des revendications 4 à 7, **caractérisé en ce que** l'élément de serrage présente un bras formant ressort (31), qui relie la surface de soutien (34) et la zone de liaison au dispositif de serrage (8, 9), et qui est mené latéralement par-delà le deuxième élément d'appui (6).

9. Dispositif de fixation selon l'une des revendications 4 à 8, **caractérisé en ce que** l'élément de serrage (31) porte une pièce de préhension (36) dépassant du dispositif de serrage (8, 9).

10. Dispositif de fixation selon l'une des revendications précédentes, **caractérisé en ce que** l'élément de serrage élastique (16) s'appuie contre le deuxième élément d'appui (6).

11. Dispositif de fixation selon la revendication 10, **caractérisé en ce que** l'élément de serrage élastique (16), lorsque le dispositif de fixation (1) est appliqué sur les parties d'os (2, 3), peut être inséré latéralement par coulissement entre le côté supérieur des parties d'os (2, 3) et le côté inférieur du deuxième élément d'appui (6), et peut à nouveau être retiré latéralement par coulissement.

12. Dispositif de fixation selon l'une des revendications 10 ou 11, **caractérisé en ce que** l'élément de serrage élastique est un ressort en étrier (16).

13. Dispositif de fixation selon la revendication 12, **caractérisé en ce que** le ressort en étrier (16) est d'une configuration en forme de U comprenant une première aile (18) formant ladite surface de soutien, et une deuxième aile (17) pouvant être appliquée contre le deuxième élément d'appui (6), ainsi qu'une branche élastique (19) reliant les deux ailes (17, 18).

14. Dispositif de fixation selon l'une des revendications 10 à 13, **caractérisé en ce que** le dispositif de serrage comprend au moins une broche (8, 9) maintenue sur le premier élément d'appui (5) et traversant le deuxième élément d'appui (6), et **en ce que** l'élément de serrage (16) est agencé des deux côtés de la broche ou des broches (8, 9).

15. Dispositif de fixation selon la revendication 14, **caractérisé en ce que** l'élément de serrage (16) présente un évidement (20) en forme de fente, dans lequel s'engagent la broche ou les broches (8, 9) du dispositif de serrage lors de l'insertion de l'élément de serrage élastique (16).

16. Dispositif de fixation selon l'une des revendications 11 à 15, **caractérisé en ce qu'**il est prévu au moins un organe d'arrêt (17a), qui assure l'arrêt de l'élément de serrage élastique (16) dans l'état inséré, à l'encontre d'une extraction, et **en ce que** l'organe d'arrêt (17a) peut être débloqué par des forces de coulissement dépassant une certaine valeur limite.

17. Dispositif de fixation selon l'une des revendications 11 à 16, **caractérisé en ce que** l'élément de serrage élastique (16), dans l'état inséré de l'élément de serrage élastique (16), porte une pièce de préhension (18a) dépassant latéralement du deuxième élément d'appui (6).
